# EUROPÄISCHE PATENTSCHRIFT

(11) **EP 3 811 910 B1**
(45) Veröffentlichungstag und Bekanntmachung des Hinweises auf die Patenterteilung: **29.11.2023**
(21) Anmeldenummer: 19204990.6
(22) Anmeldetag: 24.10.2019
(51) Int. Cl.: A61F 5/01, A61F 13/06

(54) **TEXTILTEIL**
TEXTILE PART
ÉLÉMENT TEXTILE

(43) Veröffentlichungstag der Anmeldung: 28.04.2021
(73) Patentinhaber: medi GmbH & Co. KG, 95448 Bayreuth (DE)
(72) Erfinder: Bauer, Patrick, 91275 Auerbach (DE)
(74) Vertreter: Lindner Blaumeier Patent- und Rechtsanwälte

(56) Entgegenhaltungen:
- EP-A2- 1 945 156
- DE-A1-102009 038 517
- DE-A1-102011 118 617
- DE-U1-202015 105 668
- US-A- 6 117 097
- US-A1- 2005 240 283
- US-A1- 2008 294 082

## Beschreibung

Die Erfindung betrifft ein Textilteil, insbesondere textiles orthopädisches Hilfsmittel, umfassend einen textilen Grundkörper sowie wenigstens ein am Grundkörper angeordnetes Funktionselement aus Kunststoff.

Ein solches Textilteil, wie beispielsweise aus EP 2 779 963 B1 bekannt, ist beispielsweise als Manschette oder als Bandage ausgeführt und dient der Anlage am Körper eines Trägers. Das Textilteil weist einen textilen Grundkörper auf, an dem wenigstens ein Funktionselement aus Kunststoff angeordnet ist, das beispielsweise als längliche Schiene oder als Gelenk oder Ähnliches ausgeführt ist. Dieses Funktionselement dient dazu, dem Textilteil eine bestimmte funktionale Eigenschaft zu verleihen, die dem Träger hilfreich ist, also beispielsweise stabilisierend oder versteifend wirkt, um so gegen mechanische Einwirkungen auf die von dem Textilteil übergriffene Körperpartie zu schützen und ähnliches.

Neben der Möglichkeit, ein solches Funktionselement in eine bevorzugt stricktechnisch an dem Grundkörper ausgebildete Tasche einzubringen, also beispielsweise einen Stabilisierungsstab in eine entsprechende längliche Tasche einzuschieben, ist es auch bekannt, das Funktionselement über eine stoffschlüssige Verbindung, also eine Schweißverbindung, am Grundkörper zu fixieren. Dies ist beispielsweise aus EP 2 779 963 B1 bekannt, wo ein Funktionselement über ein mit Kunststoff kaschiertes Trägerelement auf den Grundkörper, beispielsweise eine Bandage, aufgeschweißt wird. Das Funktionselement selbst ist auf das Trägerelement aufgebracht, was in einem ersten Prozessschritt beispielsweise durch Aufspritzen oder Hinterspritzen erfolgt. Dieses Kombinationsbauteil wird sodann in einem zweiten Prozessschritt auf die Bandage aufgelegt und ausschließlich im Bereich des Trägerelements, das breiter als das Funktionselement ist, aufgeschweißt, was durch Aufschmelzen der Kunststoffbeschichtung des Trägerelements erfolgt. Bei dieser Ausgestaltung ist also das Funktionselement selbst nicht mit dem Grundkörper verbunden, sondern lediglich über das Trägerelement, was dazu führt, dass es zu Einbußen in der Funktionalität des Funktionselements kommen kann, da dessen Eigenschaften, beispielsweise stabilisierende Eigenschaften, nicht unmittelbar auf den Grundkörper übertragen werden. Darüber hinaus ist auch der Herstellvorgang aufwendig, da zunächst das Kombinationsbauteil aus Funktionselement und Trägerelement in einem ersten Prozessschritt hergestellt werden muss, wonach erst in einem zweiten Prozessschritt die Applikation dieses Kombinationsbauteils am Grundkörper erfolgt.

Aus DE102009038517A1 und DE202015105668U1 sind Bandagen bekannt, an denen Funktionselemente über Formschlussverbindungen mit Befestigungselementen befestigt werden.

Der Erfindung liegt damit das Problem zugrunde, ein demgegenüber verbessertes Textilteil anzugeben. Die Erfindung ist im beigefügten Anspruchssatz beschrieben.

Zur Lösung des Problems ist bei einem Textilteil der eingangs genannten Art erfindungsgemäß vorgesehen, dass das Funktionselement formschlüssig und stoffschlüssig mit wenigstens einem Befestigungselement verbunden ist und das Befestigungselement stoffschlüssig mit dem Grundkörper verbunden ist.

Anders als im Stand der Technik sind bei dem erfindungsgemäßen Textilteil das Funktionselement und das Befestigungselement in jedem Fall formschlüssig miteinander verbunden, das heißt, dass am Funktionselement und am Befestigungselement entsprechende Formschlussgeometrien oder -konturen vorgesehen sind, die ineinandergreifen respektive miteinander in Interaktion treten und so eine formschlüssige Anordnung des Funktionselements am Befestigungselement ermöglichen. Zusätzlich ist auch eine stoffschlüssige Verbindung zwischen dem Funktionselement und dem Befestigungselement vorgesehen, das heißt, dass das Befestigungselement und das Funktionselement sowohl formschlüssig als auch stoffschlüssig miteinander verbunden sind, wozu sowohl am Funktionselement als auch am Befestigungselement entsprechende, stoffschlüssig miteinander verbindbare Materialien vorgesehen sind. Dies kann bei dem aus Kunststoff bestehenden Funktionselement das Kunststoffmaterial selbst sein, bei dem Befestigungselement beispielsweise eine entsprechende Kunststoffbeschichtung oder ein sonstiger befestigungselementseitig vorgesehener Kunststoffanteil, so dass die beiden Elemente im Rahmen eines Kunststoffschweißverfahrens oder gegebenenfalls auch im Rahmen eines Kunststoffspritzverfahrens miteinander stoffschlüssig verbunden werden können.

Da es sich bei dem Funktionselement um ein Kunststoffelement handelt, kann beispielsweise auch durch eine entsprechende Farbgebung auf die entsprechenden funktionalen respektive mechanischen Eigenschaften hingewiesen werden. So kann beispielsweise ein grün eingefärbtes Funktionselement, beispielsweise in Form einer Stabilisierungsstrebe, nur eine geringere Steifigkeit gegeben sein, während ein rot eingefärbtes Funktionselement demgegenüber steifer ist, bis hin zu beispielsweise einem blau eingefärbten Stabilisierungselement, das das steifste Funktionselement innerhalb des Sortiments ist.

Kommt sowohl ein Formschluss als auch ein Stoffschluss zum Einsatz, so ist eine besonders feste Verbindung zwischen Funktionselement und Befestigungselement gegeben, die aufgrund der formschlüssigen Verbindung in optimaler Weise eine Übertragung der funktionalen Eigenschaften des Funktionselements über das Befestigungselement auf den Grundkörper ermöglicht. Denn infolge der Formschlussverbindung kann zusätzlich zur stoffschlüssigen Verbindung beispielsweise die sich aus der Steifigkeit des Funktionselements z. B. in Form einer Stabilisierungsstrebe ergebende Abstützung respektive Immobilisierungswirkung noch besser auf den Grundkörper übertragen werden als bei nur einer stoffschlüssigen Verbindung, wie sie in einfacher Weise beispielsweise aus EP 2 779 963 B1 bekannt ist, wo das Funktionselement in Form einer ebenflächigen Stabilisierungsstrebe lediglich flächig auf dem Trägerelement aufliegt, aber selbst weder stoff- noch formschlüssig gekoppelt ist.

Bei Ausführung der Elementkombination aus Funktionselement und Befestigungselement mit form- und stoffschlüssiger Verbindung besteht darüber hinaus die Möglichkeit, diese Einheit in einem einzigen Prozessschritt am Grundkörper zu fixieren. Hierzu ist es lediglich erforderlich, zunächst das Funktionselement und das Befestigungselement formschlüssig miteinander zu verbinden und am Grundkörper anzulegen, entweder an dessen Außenseite oder Innenseite. Sodann wird durch Energiezufuhr, beispielsweise in Form von Hochfrequenz- oder Ultraschallschwingungen, das eigentliche Verschweißen der Komponenten untereinander vorgenommen. Durch diese Energiezufuhr kommt es zu einem lokalen Aufschmelzen respektive Erweichen des Kunststoffmaterials einerseits des Funktionselements, so dass es zu einer stoffschlüssigen Verbindung in den Abschnitten, in denen es am Befestigungselement anliegt, also auch im Formschlussbereich, nach dem Erkalten kommt. Im Rahmen dieses Energieeintrags kommt es andererseits auch zum Erweichen oder Aufschmelzen des Kunststoffanteils, den das Funktionselement aufweist, zumindest in den Bereichen, in denen das Befestigungselement am Grundkörper anliegt, so dass es auch dort aufgrund des Erweichens respektive Aufschmelzens dieses Kunststoffmaterials zu einem Benetzen des Grundkörpers, also dessen textiler Struktur kommt und das aufgeschmolzene Kunststoffmaterial sich stoffschlüssig mit dem Grundkörper, gegebenenfalls auch in dessen textile Struktur leicht eindringend, kommt. Das heißt, dass bei dem erfindungsgemäßen Textilteil im Rahmen eines einzigen Prozessschritts durch entsprechende Energiezufuhr zum Erwärmen und Aufschmelzen der entsprechenden Materialbereiche eine stoffschlüssige Verbindung zwischen den einzelnen Komponenten, zusätzlich zum gegebenen Formschluss, erreicht werden kann.

Wenngleich bevorzugt der Stoffschluss durch entsprechendes Verschweißen, also Kunststoffverschweißen der Komponenten erwirkt wird, indem insbesondere durch Strahlungsenergie der entsprechende Energieeintrag zum Aufschmelzen vorgenommen wird, ist es aber gleichermaßen denkbar, die Aufschmelzenergie auch in anderer Weise beispielsweise rein thermisch durch Einbringen in einen Wärmeofen oder dergleichen zu applizieren.

Das Befestigungselement und der Grundkörper sind wie beschrieben in jedem Fall stoffschlüssig miteinander verbunden, also verschweißt. Zusätzlich kann auch noch eine formschlüssige Verbindung gegeben sein, z.B. indem beide miteinander vernäht werden, wobei hier der Faden den Formschluss erwirkt. Auch ein Vernieten ist denkbar.

Das Befestigungselement selbst kann entweder flexibel oder starr sein. Unter "flexibel" wird eine entsprechende Flexibilität des Befestigungselements verstanden, die es ermöglicht, dass das Befestigungselement grundsätzlich entsprechenden Bewegungen des Grundkörpers respektive entsprechenden Geometrieänderungen des Grundkörpers in ausreichendem Umfang folgen kann, also beispielsweise, wenn sich der Grundkörper einem darunter befindlichen Körperbereich wie einem Unterschenkel oder Ähnlichem anpasst, diese Anpassung ohne weiteres mitvollziehen kann. Das Befestigungselement ist also weit flexibler als das Funktionselement beispielsweise dann in Form einer Stabilisierungsstrebe oder dergleichen. Das Befestigungselement kann in einem solchen Fall quasi folienartig mit einer entsprechenden Wandstärke von beispielsweise 0,5 - 1,5 mm sein, wobei beispielsweise im Bereich der entsprechenden Formschlussgeometrien entsprechend größere Wandstärken vorgesehen sein können, um eine entsprechende Formschusshalterung zu gewährleisten. Auch kann es sich um einen textilen Träger mit einer Kunststoffbeschichtung handeln, der ebenfalls sehr flexibel ist. Unter "starr" wird demgegenüber eine mechanische Eigenschaft des Befestigungselements verstanden, die den mechanischen Eigenschaften des Funktionselements zumindest ähnlich ist oder nahekommt. Das heißt, dass auch das Befestigungselement selbst eine hinreichende Steifigkeit aufweist und von sich aus bereits den Grundkörper, wie beschrieben bevorzugt ein textiler Gestrickkörper, hinreichend aussteift. Auch hier kann das Befestigungselement aus Kunststoff sein, weist dann jedoch entweder eine entsprechend größere Dicke auf, beispielsweise im Bereich von 1,5 - 2,5 mm, oder ist aus einem entsprechend steifen respektive weniger flexiblen Kunststoffmaterial gefertigt. Das heißt, dass grundsätzlich auch durch Wahl der Ausgestaltung des Befestigungselements beispielsweise einem therapeutischen Zweck, den das Textilteil respektive das Funktionselement erfüllen soll, Rechnung getragen werden kann, da das Befestigungselement den Zweck unterstützend ausgelegt werden kann.

Wie beschrieben besteht die Möglichkeit, dass das Befestigungselement ein Kunststoffelement ist, ebenso wie auch das Funktionselement. Alternativ kann das Befestigungselement auch einen Träger mit einer ein- oder beidseitigen Kunststoffbeschichtung aufweisen. Bei dem Träger kann es sich beispielsweise um ein Textil handeln, beispielsweise ein Velours, gegebenenfalls auch mit Kletteigenschaften, das heißt, dass an ihm Elemente über eine Klettverbindung zusätzlich befestigt werden können, was die Funktionalität der Einheit aus Funktionselement und Befestigungselement noch weiter erhöht. Bei einem Velours respektive Klettvelour handelt es sich um eine mittelfeine Wirkware, bei der Einzelfilamente von Fadenflottungen durch einen Ausrüstungsprozess wie Aufrauen insbesondere auf einer Walzen-Kratzen-Raumaschine zu Schlingen aufgestellt und verfestigt werden, um klettfähig zu sein. Die Erfindung ist jedoch nicht auf die Verwendung eines solchen Velours beschränkt, vielmehr kann jedes Textil respektive jede textile Schicht in Form eines Gewebes, Gewirkes oder Gestricks als Befestigungselement verwendet werden. Dieser Träger ist je nachdem, ob eine stoffschlüssige Verbindung nur zum Grundkörper oder auch zum Funktionselement erfolgen soll, entweder einseitig nur an seiner Unterseite, die zum Grundkörper hinweist, mit einer Kunststoffbeschichtung versehen, oder beidseitig, also auch zumindest in den Bereichen, in denen ein Stoffschluss zum Funktionselement erwirkt werden soll. Diese Kunststoffbeschichtung kann beispielsweise auf den Träger, also beispielsweise den textilen Träger, aufkaschiert oder aber auch aufgespritzt sein.

Neben der Ausrüstung des Befestigungselements selbst mit der Kunststoffbeschichtung ist es aber auch denkbar, die aufschmelzbare Kunststoffebene durch Zwischenschaltung einer entsprechenden, die stoffschlüssige Verbindung erwirkenden Zwischenlage zwischen Befestigungselement und Grundkörper zu erwirken, das heißt, dass eine entsprechende flächige, aufschmelzbare Kunststofffolie oder Ähnliches zwischengelegt wird, die dann über den Energieeintrag entsprechend aufgeschmolzen wird, so dass sich sowohl das Befestigungselement, also dann der verbleibende Träger desselben, wie auch der Grundkörper mit der aufgeschmolzenen Zwischenlage stoffschlüssig verbinden.

Wird der Stoffschluss durch Verschweißen, also Kunststoffschweißen erwirkt, so sind natürlich die entsprechenden Materialien der hierüber miteinander zu verbindenden Kunststoffelemente, also des Funktionselements und des Befestigungselements so zu wählen, dass sie sich stoffschlüssig verbinden können, mithin also chemisch insoweit kompatibel sind. Grundsätzlich kann das Funktionselement und/oder das Befestigungselement oder die Kunststoffbeschichtung oder die Zwischenlage aus Polyamid, Polyvinylchlorid, Polyurethan, Polyethylen oder einem thermoplastischen Elastomer, insbesondere auf Urethanbasis, bestehen. Diese Aufzählung ist jedoch nicht abschließend, es sind durchaus auch weitere Kunststoffe verwendbar, die entsprechende Eigenschaften aufweisen, entweder was das Verschweißen angeht, oder die entsprechende mechanische Eigenschaften zeigen.

Grundsätzlich besteht die Möglichkeit, an einem Befestigungselement nur ein Funktionselement anzuordnen, also beispielsweise nur eine Stabilisierungsschiene oder Ähnliches. Je nach Art des Textilteils und/oder je nach Art der therapeutischen Aufgabe kann es aber auch erforderlich sein, an einem gemeinsamen Befestigungselement mehrere Funktionselemente entweder formschlüssig oder formschlüssig und stoffschlüssig anzuordnen. Beispielsweise können zwei oder mehr Stabilisierungsschienen nebeneinander angeordnet sein, die parallel oder unter einem Winkel zueinander verlaufen können, oder die unterschiedliche Formen oder Längen aufweisen etc. Das heißt, dass hierüber in relativ umfangreicher Weise eine Variationsmöglichkeit besteht, um sich in bestmöglicher Weise einer therapeutische Aufgabe anzupassen. Dabei ist es auch denkbar, die Befestigungsweise der mehreren Funktionselemente unterschiedlich zu gestalten. Beispielsweise wird ein zentrales Funktionselement, das zwingend anzuordnen ist, formschlüssig und stoffschlüssig angeordnet, also nicht austauschbar, während ein oder mehrere weitere Funktionselemente beispielsweise nur formschlüssig angebracht sind, so dass sie bei Bedarf entnommen oder gegen andere ausgetauscht werden können etc.

Weiterhin ist es denkbar, dass ein aus mehreren relativ zueinander bewegbaren Teilen bestehendes Funktionselement vorgesehen ist, wobei ein Teil mit einem ersten Befestigungselement und ein weiteres Teil mit einem zweiten Befestigungselement formschlüssig und stoffschlüssig verbunden ist und beide Befestigungselemente am Grundkörper stoffschlüssig befestigt sind. Bei dieser Ausgestaltung wird als Funktionselement ein gelenkartiges Funktionselement verwendet, das zwei Gelenkschenkel aufweist, die über eine Gelenkverbindung schwenkbar miteinander verbunden sind. Jeder Gelenkschenkel ist an bzw. in einem separaten Befestigungselement fixiert, wobei beide Befestigungselemente stoffschlüssig am Grundkörper befestigt sind. Grundsätzlich besteht auch hier die Möglichkeit, zu dem Gelenk-Funktionselement auch noch ein weiteres Funktionselement wie beispielsweise eine dann relativ kurze Stabilisierungsstrebe oder Ähnliches am ersten und/oder zweiten Befestigungselement in den beiden alternativen Befestigungsvarianten anzuordnen.

Ein zentrales Merkmal der Erfindung ist die formschlüssige Befestigung des Formschlusselements am Befestigungselement. Hierzu kann am Befestigungselement wenigstens eine einseitig hinterschnittene Nut vorgesehen sein, oder eine beidseits hinterschnittene Nut. Im Falle einer einseitig hinterschnittenen Nut weist das Funktionselement eine entsprechende, formkompatible Formschlussgeometrie auf, es weist also einen entsprechenden Falz an einer Seite oder eine Nut auf, so dass ein entsprechender Eingriff möglich ist. Das Formschlusselement hat in diesem Fall also quasi eine L- oder C-Form. Im Falle einer einseitig hinterschnittenen Nut kann an der dem hinterschnittenen Nutschenkel gegenüberliegenden Seite ein gerader Nutschenkel vorgesehen sein, alternativ kann hier das Funktionselement auch freiliegen.

Im Falle einer beidseits hinterschnittenen Nut ist das Funktionselement beidseits formschlüssig fixiert und weist eine T-Form mit beidseitigen Falzen oder eine Doppel-T-Form mit beidseitigen Nuten für den Eingriff auf.

Alternativ ist es auch denkbar, dass am Befestigungselement wenigstens ein T-förmiger Vorsprung ausgebildet ist, der in einer beidseits hinterschnittenen Nut am Funktionselement aufgenommen ist. Hier wird das Befestigungselement quasi in die funktionselementseitige Nut eingeschnappt, worüber der Stoffschluss erwirkt wird. Alternativ kann am Befestigungselement auch ein L-förmiger Vorsprung ausgebildet sein, der in eine nur einseitig hinterschnittenen Nut am Funktionselement eingreift. Auch hierüber kann eine entsprechende Formschlussverbindung realisiert werden.

Grundsätzlich zeichnen sich die Formschlussverbindungen also immer über einen entsprechenden Ein- oder Übergriff aus, über den die Fixierung erfolgt.

In Weiterbildung der Erfindung ist es zweckmäßig, wenn am Befestigungselement eine oder mehrere Durchbrechungen vorgesehen sind. Durch diese Durchbrechungen wird die Auflagefläche des Befestigungselements am Grundkörper reduziert, das heißt, dass die Befestigungsfläche und damit auch die Verbindungsebene, über die das Befestigungselement stoffschlüssig mit dem Grundkörper verbunden ist, reduziert ist. Der Grundkörper, also beispielsweise der textile Gestrickkörper, bleibt hierdurch etwas flexibler, was insbesondere bei einem flexibleren Befestigungselement, das ja gerade etwaige Geometrieänderungen des Grundkörpers mitvollziehen soll, zweckmäßig ist. Wenngleich die Möglichkeit besteht, die einzelnen Komponenten, also Funktionselement, Befestigungselement und Grundkörper als separate Elemente zu verwenden und in dem bevorzugt einstufigen Verfahren untereinander zu fixieren, ist es in einer Weiterbildung der Erfindung aber auch denkbar, dass das oder die Funktionselemente und das oder die Befestigungselemente in einem Kunststoffspritzverfahren, insbesondere einem 2K-Kunststoffspritzverfahren gefügt sind. Das ohnehin aus Kunststoff bestehende Funktionselement und das Befestigungselement, egal ob dieses ein Vollkunststoff-Bauteil ist oder einen Träger mit einer Kunststoffbeschichtung aufweist, können immer in einem Kunststoffspritzverfahren gefügt werden, das heißt, dass beide gleichzeitig gespritzt und dabei gefügt werden, also formschlüssig miteinander gekoppelt werden. Auch ist es denkbar, dass das Funktionselement auf den die Kunststoffbeschichtung aufweisenden Träger in einem Kunststoffspritzverfahren aufgespritzt und damit formschlüssig gefügt wird.

Der Grundkörper selbst ist, wie beschrieben, ein Gestrickkörper, wobei insbesondere ein kompressiver Gestrickkörper verwendet wird. Ein kompressiver Gestrickkörper übt eine definierte Kompression, also Kraft auf die darunterliegende, übergriffene Körperpartie respektive das Gewebe aus. Lediglich exemplarisch wird bezüglich eines solchen kompressiven Textilteils in Form medizinischer Kompressionsstrümpfe auf die Definitionen in "Gütesicherung RAL-GZ387/1 Medizinische Kompressionsstrümpfe" sowie betreffend medizinische Kompressionsarmstrümpfe auf "Gütesicherung RAL-GZ387/2 Medizinische Kompressionsarmstrümpfe" der Gütezeichengemeinschaft medizinische Kompressionsstrümpfe e.V. verwiesen, wo entsprechende Güte- und Prüfbestimmungen für medizinische Kompressions(arm)strümpfe, die ebenfalls ein erfindungsgemäßes Textilteil sein können, angegeben sind.

Bei dem Textilteil selbst kann es sich um unterschiedliche Typen handeln. Es kann sich um eine Bandage, eine Orthese oder einen Strumpf, insbesondere einen Kompressionsstrumpf, handeln, an der oder dem ein längliches oder gebogenes stabartiges Funktionselement, ein gelenkartiges Funktionselement, ein als Gurtdurchführung ausgebildetes Funktionselement, ein als manuell greifbare Zughilfe ausgebildetes Funktionselement oder ein als Pelotte ausgebildetes Funktionselement vorgesehen ist. Hier sind also unterschiedlichste Variationsmöglichkeiten hinsichtlich der Ausgestaltung des Textilteils und dessen therapeutischen Zweck gegeben, wie auch unterschiedlichste Funktionselemente verwendet werden können.

Neben dem Textilteil selbst betrifft die Erfindung ferner ein Verfahren zur Herstellung eines solchen Textilteils. Dieses zeichnet sich dadurch aus, dass auf einem textilen Grundkörper wenigstens ein Funktionselement, das formschlüssig und stoffschlüssig mit wenigstens einem Befestigungselement verbunden ist, angeordnet wird, wonach durch Energiezufuhr zwischen dem Befestigungselement und dem Grundkörper eine stoffschlüssige Verbindung erwirkt wird.

Daneben wird auch durch die Energiezufuhr eine stoffschlüssige Verbindung zwischen dem Funktionselement und dem Befestigungselement erwirkt, das heißt, dass dort nicht nur die formschlüssige Verbindung, sondern zusätzlich auch ein stoffschlüssiger Verbund erzeugt wird.

Das Erwirken der stoffschlüssigen Verbindung erfolgt erfindungsgemäß bevorzugt dadurch, dass das Befestigungselement oder das Befestigungselement und das Funktionselement oder eine zwischen dem Befestigungselement und dem Grundkörper angeordnete Zwischenlage zur Erwirkung der Verbindung lokal aufgeschmolzen wird. Die Energiezufuhr erfolgt bevorzugt durch Strahlungsenergie, insbesondere durch Hochfrequenz- oder Ultraschallschwingungen, das heißt, dass letztlich ein Kunststoffschweißverfahren verwendet wird, um den stoffschlüssigen Verbund zu erwirken, nachdem die entsprechenden aufschmelzbaren Komponenten allesamt aus Kunststoff sind oder zumindest einen aufschmelzbaren Kunststoffanteil aufweisen.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: eine Prinzipdarstellung in Form einer Explosionsansicht eines erfindungsgemäßen Texteilteils mit separatem Funktionselement, separatem Befestigungselement mit separatem Grundkörper,
- Fig. 2: die Anordnung aus Fig. 1 mit auf den Grundkörper aufgesetztem Befestigungselement mit daran bereits formschlüssig fixiertem Funktionselement,
- Fig. 3: die Anordnung aus Fig. 2 im verschweißten Endzustand,
- Fig. 4: eine Prinzipdarstellung einer zweiten Ausführungsform eines erfindungsgemäßen Textilteils in Form einer Teilansicht,
- Fig. 5: eine Prinzipdarstellung einer dritten Ausführungsform eines erfindungsgemäßen Textilteils in einer Teilansicht,
- Fig. 6: eine Prinzipdarstellung einer vierten Ausführungsform eines erfindungsgemäßen Textilteils in einer Teilansicht,
- Fig. 7: eine Prinzipdarstellung einer ersten konkreten Realisierungsform eines Textilteils in Form einer Bandage,
- Fig. 8: eine Prinzipdarstellung einer zweiten konkreten Realisierungsform eines Textilteils in Form einer Orthese,
- Fig. 9: eine Prinzipdarstellung einer dritten konkreten Realisierungsform eines Textilteils in Form eines Strumpfes, und
- Fig. 10: eine vierte konkrete Ausgestaltung eines Textilteils in Form eines Strumpfes mit einer Gurtführung.

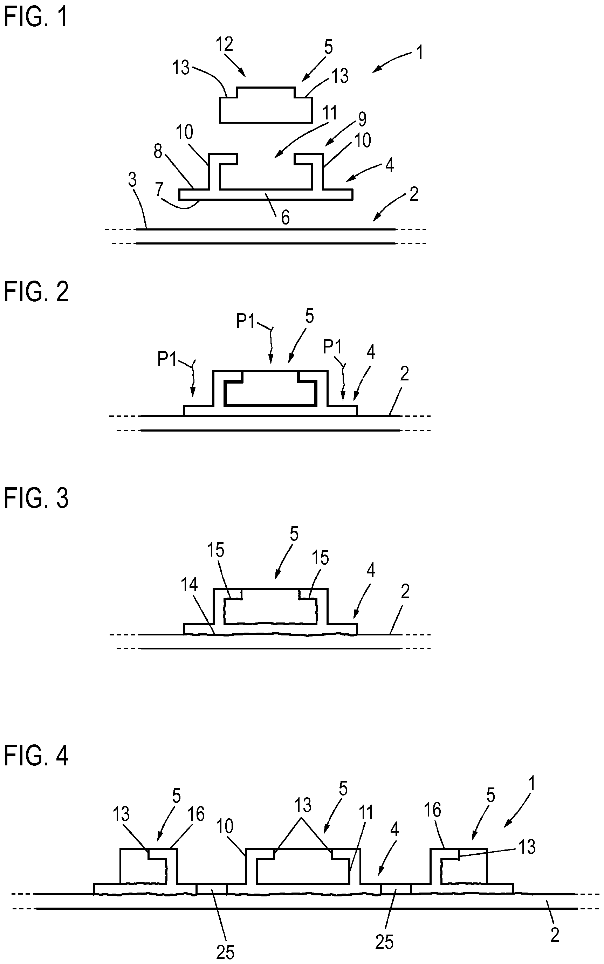

Fig. 1 zeigt in Form einer Explosionsansicht ein erfindungsgemäßes Textilteil 1, umfassend einen Grundkörper 2, bevorzugt ein textiler Gestrickkörper, insbesondere mit kompressiven Eigenschaften. Der Grundkörper 2 weist eine Außenseite 3 auf, auf die nachfolgend ein Befestigungselement 4 sowie ein Funktionselement 5, das am Befestigungselement 4 fixiert ist, aufgesetzt werden. Alternativ zur Anordnung an der Außenseite 3 besteht natürlich auch die Möglichkeit, Funktionselement 5 und Befestigungselement 4 an der gegenüberliegenden Innenseite des Grundkörpers 2 aufzusetzen.

Das Befestigungselement 2 ist vorliegend beispielsweise ein Kunststoffelement, beispielsweise nach Art einer länglichen, flexibleren oder eher steiferen Kunststofffolie, die eine entsprechende Elementbasis 6 mit einer Unterseite 7 aufweist, wobei an der gegenüberliegenden Oberseite 8 im gezeigten Beispiel eine Formschlusskontur 9 ausgebildet ist, umfassend zwei Schenkel 10, die eine beidseits hinterschnittene Nut 11 definieren, in die das formkompatibel ausgeführte Funktionselement 5 eingesetzt respektive eingeschnappt werden kann.

Hierzu weist das ebenfalls aus Kunststoff bestehende Funktionselement 5 ebenfalls eine entsprechende beidseitige Formschlusskontur 12 umfassend zwei Falze 13 auf, in die die querverlaufenden Abschnitte der Schenkel 10 in der Einschnappstellung, siehe Fig. 2, eingreifen.

Das Funktionselement 5, beispielsweise eine Stabilisierungsschiene, ist wie beschrieben ebenfalls aus Kunststoff. Es kann sich dabei um denselben Kunststoff wie den des Befestigungselements 4 handeln, oder um einen anderen Kunststoff, solange beide miteinander in einem Kunststoffschweißverfahren stoffschlüssig miteinander verbunden werden können.

Auch können das Befestigungselement 4 und das Funktionselement 5 gleiche Farbe aufweisen, alternativ ist es aber auch denkbar, dass das Funktionselement 5 eine andere Farbe aufweist als das Befestigungselement. Beispielsweise ist bei einem schwarzen Grundkörper 2, also einem schwarzen textilen Gestrickkörper, auch das Befestigungsnelement schwarz ausgeführt, damit es möglichst unauffällig ist. Demgegenüber kann das Funktionselement 5 eine deutlich andere Farbe aufweisen, wobei diese andere Farbe einerseits aus Designgründen gewählt werden kann, andererseits aber auch, um hierüber anzuzeigen, welche mechanischen Eigenschaften das Funktionselement 5 aufweist, also beispielsweise wie steif es ist, wenn es sich bei dem Funktionselement 5 um eine Stabilisierungsstrebe handelt.

Im nächsten, in Fig. 2 gezeigten Schritt wird zunächst das Funktionselement 5 in die Nut 11 des Befestigungselements 4 eingeschnappt oder eingerastet, so dass es formschlüssig hierin fixiert und aufgenommen ist. Über diesen Formschluss ist eine sichere Befestigung gegeben, wie hierüber auch sichergestellt ist, dass die mechanischen Eigenschaften, also die funktionalen Eigenschaften des Funktionselements 5, zwingend auf das Befestigungselement 4 übertragen werden. Nachdem das Befestigungselement 4, worauf nachfolgend noch eingegangen wird, fest mit dem Grundkörper 2 verbunden wird, werden diese Eigenschaften hierüber dann auch zwangsläufig auf den Grundkörper 2 und damit auch auf das hiervon übergriffene Körperteil übertragen.

Ersichtlich sitzt, siehe Fig. 2, auch das Befestigungselement 4 mit seiner Unterseite 7 auf der Außenseite 3 des Grundkörpers 2 auf, es ist also eine großflächige Auflage gegeben.

Wenngleich in den Figuren 1 und 2 das Befestigungselement 4 und das Funktionselement 5 als separate Bauteile gezeigt sind, ist festzuhalten, dass beide auch bereits werks- oder herstellungsseitig als 2K-Bauteil in einem 2K-Spritzgussverfahren hergestellt werden können, so dass beide nach dem Spritzgießen bereits formschlüssig miteinander verbunden sind. Ein separates Fügen, wie in den Fig. 1 und 2 gezeigt, ist dann nicht erforderlich, vielmehr kann das 2K-Bauteil aus Befestigungselement und Funktionselement direkt auf den Grundkörper gelegt und anschließend verschweißt werden, was nachfolgend im nächsten, ebenfalls in Fig. 2 gezeigten Schritt erläutert wird.

Im nächsten Schritt, ebenfalls in Fig. 2 dargestellt, wird nun eine stoffschlüssige Verbindung zumindest zwischen dem Befestigungselement 4 und dem Grundkörper 2 erwirkt, im gezeigten Beispiel aber auch zwischen dem Funktionselement 5 und dem Befestigungselement 4. Zur Erwirkung der stoffschlüssigen Schweißverbindung wird in einem einzigen Prozessschritt Energie zugeführt, wie durch die Pfeile P1 dargestellt ist. Bevorzugt wird die Energie in Form von Hochfrequenz- oder Ultraschallschwingungen zugeführt, also ein Kunststoffschweißen vorgenommen. Die Energiezufuhr bewirkt, dass es zu einem lokalen oberflächlichen Aufschmelzen des Befestigungselements im Bereich seiner Anlage zum Grundkörper, also im Bereich seiner Unterseite 7 kommt. Hierdurch kommt es zu einer Benetzung der Oberfläche des Grundkörpers 2 durch das aufgeschmolzene Kunststoffmaterial, dieses dringt gegebenenfalls auch etwas in die textile Struktur des Grundkörpers 2 ein, so dass es nach Aushärten des aufgeschmolzenen Kunststoffmaterials zu einem festen, stoffschlüssigen Verbund kommt.

Im gezeigten Beispiel wird auch eine stoffschlüssige Verbindung zwischen dem Funktionselement 5 und dem Befestigungselement 4 im Bereich der Formschlussverbindung realisiert. Das heißt, dass durch den Energieeintrag auch ein Aufschmelzen des Materials des Befestigungselements 4 wie auch des Funktionselements 5 im jeweiligen benachbarten Grenzflächenbereich erfolgt, so dass nach dem Aushärten auch dort eine stoffschlüssige Verbindung gegeben ist.

Der fertige fixierte Endzustand ist in Fig. 3 gezeigt. Ersichtlich ist, wie durch die geriffelte Linie dargestellt ist, eine stoffschlüssige Verbindung 14 zwischen dem Befestigungselement 4 und dem Grundkörper 2 gegeben, wie auch, ebenfalls durch die geriffelten Linien dargestellt ist, stoffschlüssige Verbindungen 15 zwischen dem Funktionselement 5 und dem Befestigungselement 4 im Nutbereich gegeben sind.

Fig. 4 zeigt eine weitere Alternative eines Textilteils 1, wobei für gleiche Elemente gleiche Bezugszeichen verwendet werden. Vorgesehen sind wiederum ein Befestigungselement 4 sowie im gezeigten Beispiel drei Funktionselemente 5 und ein Grundkörper 2, wiederum bevorzugt ein textiler Gestrickkörper. An dem Befestigungselement 4 sind vorliegend drei unterschiedliche Befestigungsstellen gegeben, nämlich eine mittige Befestigungsstelle, die der aus den Figuren 1 - 3 entspricht, realisiert über die beiden L-förmigen Schenkel 10, die die beidseits hinterschnittene Nut 11 definieren. In diese ist das die beiden Falze 13 aufweisende Funktionselement bereits formschlüssig eingeschnappt.

Des Weiteren sind zwei Schenkel 16 vorgesehen, die jeweils L-förmig ausgeführt sind, jedoch alleinstehend angeordnet sind. Ihnen zugeordnet und an ihnen formschlüssig fixiert sind zwei weitere Funktionselemente 5, die eine umgekehrte Formschlusskontur in Form jeweils eines Falzes 13 aufweisen. Sie sind quasi unter den jeweiligen L-förmigen Schenkel 16 geschoben, so dass dieser in den Falz 13 mit seinem Querschenkel eingreift. Dort ist ein Formschluss gegeben. Um die Funktionselemente 5 zusätzlich zu fixieren, wurde im Bereich dieser beiden seitlichen Funktionselemente 5 Energie zugeführt, so dass dort entsprechende stoffschlüssige Verbindungen 15 ausgebildet sind, wie natürlich auch eine entsprechende stoffschlüssige Verbindung 14 zwischen dem Befestigungselement 4 und dem Grundkörper 2 über den Energieeintrag erwirkt wird. Fig. 4 dient als Prinzipdarstellung, dass neben einem Kombinationsverbund aus stoffschlüssigem Verbund und formschlüssigen Verbund auch nur ein reiner Formschlussverbund gegeben sein kann.

Gezeigt sind hier des Weiteren Durchbrechungen 25, die am Befestigungselement ausgebildet sind. Über diese wird die Auflagefläche zwischen Befestigungselement 4 und Grundkörper 2 etwas verringert, so dass die Flexibilität oder Elastizität des Grundkörpers 2 durch das Anschweißen des Befestigungselements 4 etwas weniger beeinträchtigt wird, was insbesondere dann zweckmäßig ist, wenn es sich bei dem Befestigungselement 4 selbst um ein flexibles Element handelt.

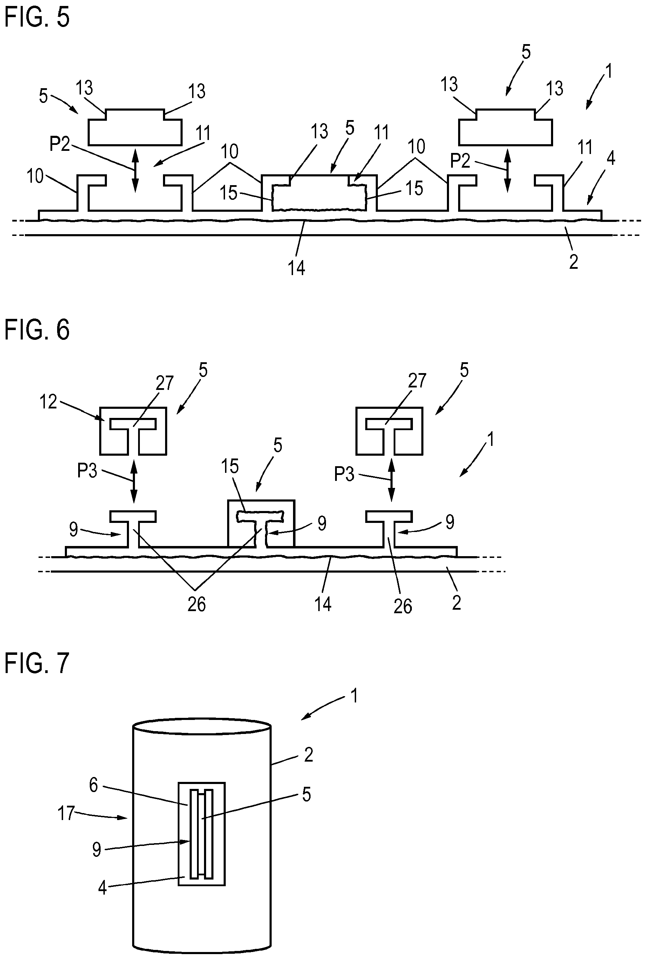

Ein drittes Ausführungsbeispiel eines erfindungsgemäßen Textilteils 1 zeigt Fig. 5. Vorgesehen ist wiederum der Grundkörper 2 sowie das Befestigungselement 4, an dem wiederum drei Aufnahmen für drei Funktionselemente 5 vorgesehen sind, die jeweils zwei L-förmige Schenkel 10 aufweisen, die jeweils eine beidseits hinterschnittene Nut 11 definieren. Die drei Funktionselemente 5 (es können natürlich, wie auch in den anderen Beispielen, auch nur zwei oder mehr als drei vorgesehen sein) weisen jeweils eine formkompatible T-förmige Geometrie mit jeweils zwei Falzen 13 auf, so dass sie, wie am mittleren Funktionselement 5 gezeigt formschlüssig in die jeweilige Nut 11 eingreifen können.

Bei dem gezeigten Ausführungsbeispiel ist, neben der stoffschlüssigen Schweißverbindung 14 zwischen dem Befestigungselement 4 und dem Grundkörper 2, wiederum eine stoffschlüssige Verbindung 15 zwischen dem mittleren Funktionselement 5 und dem Befestigungselement 4 respektive dessen Schenkeln 10 realisiert, wie über die geriffelten Linien dort gezeigt ist. Die beiden benachbart dazu vorgesehenen Funktionselemente 5 sind in die entsprechenden Nuten 11 einsetzbar, können aus diesen aber auch entnommen werden, wie durch die Doppelpfeile P2 dargestellt ist. Das heißt, dass für den Anwender die Wahlmöglichkeit besteht, zusätzlich zum mittigen Funktionselement 5 eines oder beide benachbarten Funktionselemente einzusetzen und dadurch die Eigenschaften des Textilteils 1 zu variieren.

Handelt es sich bei den Funktionselementen 5 beispielsweise um Stabilisierungsstreben, so weist das Textilteil über die mittige Stabilisierungsstrebe eine erste Grundstabilisierung auf. Diese kann erhöht werden, wenn ein oder beide zusätzlichen Funktionselemente 5, also Stabilisierungsstreben, eingeschnappt werden. Bei Bedarf können sie dann auch wieder, da nur ein Formschluss gegeben ist, entnommen werden.

Die entsprechenden Funktionselemente 5 respektive Stabilisierungsstreben können dabei parallel zueinander verlaufen oder unter einem Winkel zueinander, sie können unterschiedliche Längen aufweisen oder aber auch unterschiedliche mechanische Steifigkeiten etc., so dass eine große Variationsbandbreite gegeben ist.

Fig. 6 zeigt eine Ausgestaltung eines Textilteils 1 mit einem Grundkörper 2, wiederum vorzugsweise ein textiler Gestrickkörper, einem Befestigungselement 4 sowie auch hier drei Funktionselementen 5. Auch hier sind entsprechende Formschlusskonturen 9, 12 an dem Befestigungselement 4 sowie an den Funktionselementen 5 ausgebildet. Anders als die hinterschnittenen Nuten am Befestigungselement 4 sind hier am Befestigungselement 4 T-förmige Formschlusskonturen 9 ausgebildet, die sich beispielsweise als längliche T-förmige Schienen längs des Befestigungselements 4 erstrecken. Die Formschlusskontur 9 ist als T-förmiger Vorsprung 26 ausgeführt.

Jedes Funktionselement 5 weist eine entsprechende Formschlusskontur 12 auf, mit der es auf die T-förmigen Formschlusskonturen 9 aufgeschnappt werden kann, wie im Falle des mittleren Funktionselements 5 gezeigt. An diesem ist auch wiederum eine entsprechende stoffschlüssige Verbindung 15, wie durch die geriffelte Linie angedeutet ist, erwirkt, das heißt, dass dort neben dem Formschluss zusätzlich auch ein nicht lösbarer Stoffschluss gegeben ist. Die Formschlusskontur 12 ist als T-förmige, beidseits hinterschnittene Nut 27.

Die beiden benachbarten Funktionselemente 5 können jedoch nach Bedarf, siehe die Doppelpfeile P3, angeordnet oder entfernt werden, wozu sie lediglich auf die entsprechenden T-förmigen Formschlusskonturen 9 aufzuschnappen sind.

Auch bei diesem Ausführungsbeispiel besteht, ähnlich wie bei dem gemäß Fig. 4, eine große Variationsbandbreite hinsichtlich der Auslegung der Eigenschaften des Textilteils insbesondere im Hinblick auf die zu erfüllende therapeutische Aufgabe. Es kann sich um unterschiedlich steife Funktionselemente handeln, oder gleiche mechanische Eigenschaften aufweisende Funktionselemente. Sie können sich in ihrer Länge unterscheiden, wie sie auch parallel oder unter einem Winkel zueinander verlaufen können etc. Natürlich ist auch hier, wie durch die geriffelte Linie dargestellt ist, die stoffschlüssige Verbindung 14 des Befestigungselements 4 zum Grundkörper 2 gegeben, die bei allen Erfindungsvarianten vorgesehen ist.

Fig. 7 zeigt ein erstes konkretes Realisierungsbeispiel eines Textilteils 1 in Form einer Bandage 17. Gezeigt ist der Grundkörper 2, bevorzugt wiederum ein textiler Grundkörper in Form eines Gestricks, Gewebes oder Gewirkes. Bevorzugt wird ein kompressives Gestrick in Form eines Rund- oder Flachgestricks verwendet. Gezeigt ist des Weiteren das Befestigungselement 4, das stoffschlüssig an der Außenseite 3 des Grundkörpers 2 angeschweißt ist, wie auch ein Funktionselement 5, das hier an der Formschlusskontur 9 des Befestigungselements 4 aufgenommen ist, also in der beidseits hinterschnittenen Nut eingeschnappt ist. In dieser ist es formschlüssig fixiert, wobei hier, nachdem die Formschlusskontur 9 beidseits offen ist, die Fixierung eine Klemmfixierung ist. Die Anordnung entspricht also der wie in den Figuren 1 - 3 gezeigt.

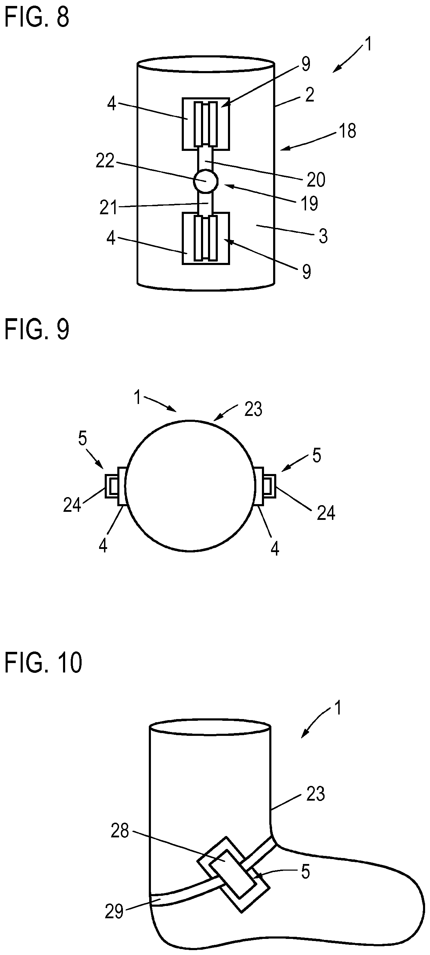

Fig. 8 zeigt ein Textilteil 1 in Form einer Orthese 18. An dieser sind zwei Befestigungselemente 4 stoffschlüssig fixiert, an denen ein gemeinsames Funktionselement 5 hier in Form eines Gelenks 19 angeordnet ist. Das Gelenk 19 besteht aus zwei Gelenkstreben 20, 21, die über eine Gelenkverbindung 22 schwenkbar miteinander verbunden sind. Jeder Gelenkschenkel 20, 21 ist in einer entsprechenden Formschlusskontur 9, die beispielsweise wiederum der gemäß der Figuren 1 - 3 entspricht, formschlüssig aufgenommen, und auch stoffschlüssig fixiert.

Fig. 9 zeigt schließlich ein Textilteil 1 in Form eines Strumpfes 23, vorzugsweise eines Kompressionsstrumpfes. Auch hier ist am Grundkörper 2 beidseits jeweils ein Befestigungselement 4 und an diesem form- und stoffschlüssig ein Funktionselement 5 hier in Form eines manuell zu greifenden Griffs 24 angeordnet, welche Griffe 24 als Anziehhilfe zum Hochziehen des Strumpfes 23 dienen. Da hier eine relativ große Kraft zu übertragen ist, sind die Griffe 24 form- und stoffschlüssig fixiert.

Fig. 10 zeigt schließlich ein Textilteil 1 in Form eines Strumpfes 23, an dem mittels des Befestigungselements 4 ein Funktionselement 5 in Form eines eine Durchbrechung aufweisenden Gurtführungsbauteils 28 angeordnet ist, durch das ein Gurt 29 geführt ist, über den eine Zügelung erfolgen kann.

Die Erfindung ist nicht auf die gezeigten Ausführungsbeispiele beschränkt, weder was die Ausgestaltung der Formschlusskonturen noch deren Anordnung und Anzahl angeht, ebenso wenig bezüglich der konkreten Realisierungsvarianten in Form von Bandage, Orthese oder Strumpf. Vielmehr sind auch andere Ausgestaltungen denkbar. Die Erfindung ist in den Ansprüchen definiert.

## Patentansprüche

1. Textilteil, insbesondere textiles orthopädisches Hilfsmittel, umfassend einen textilen Grundkörper (2) sowie wenigstens ein am Grundkörper (2) angeordnetes Funktionselement (5) aus Kunststoff, wobei das Funktionselement (5) formschlüssig und stoffschlüssig mit wenigstens einem Befestigungselement (4) verbunden ist, wobei zur Erwirkung der Formschlussverbindung am Funktionselement (5) und am Befestigungselement (4) entsprechende, ineinander ein- oder einander übergreifende Formschlusskontouren (9, 12) vorgesehen sind, und das Befestigungselement (4) stoffschlüssig mit dem Grundkörper (2) verbunden ist.

2. Textilteil nach Anspruch 1, **dadurch gekennzeichnet, dass** das Befestigungselement (4) flexibel oder starr ist.

3. Textilteil nach Anspruch 1 oder 2, **dadurch gekennzeichnet, dass** das Befestigungselement (4) ein Kunststoffelement ist oder einen Träger mit einer ein- oder beidseitigen Kunststoffbeschichtung aufweist, oder dass zwischen dem Befestigungselement (4) und dem Grundkörper (2) eine die stoffschlüssige Verbindung erwirkende, aufgeschmolzene Zwischenlage vorgesehen ist.

4. Textilteil nach einem der vorangehenden Ansprüche, **dadurch gekennzeichnet, dass** das Funktionselement (5) und/oder das Befestigungselement (4) oder die Kunststoffbeschichtung oder die Zwischenlage aus Polyamid, Polyvinylchlorid, Polyurethan, Polyethylen oder einem thermoplastischen Elastomer besteht.

5. Textilteil nach einem der vorangehenden Ansprüche, **dadurch gekennzeichnet, dass** an einem gemeinsamen Befestigungselement (4) weitere Funktionselemente (5) entweder formschlüssig oder formschlüssig und stoffschlüssig angeordnet sind.

6. Textilteil nach einem der vorangehenden Ansprüche, **dadurch gekennzeichnet, dass** das Funktionselement (5) ein aus mehreren relativ zueinander bewegbaren Teilen bestehendes Funktionselement (5, 19) ist, wobei ein Teil mit einem ersten Befestigungselement (4) und ein weiteres Teil mit einem zweiten Befestigungselement (4 ) formschlüssig und stoffschlüssig verbunden ist und beide Befestigungselemente (4) am Grundkörper (2) stoffschlüssig befestigt sind.

7. Textilteil nach einem der vorangehenden Ansprüche, **dadurch gekennzeichnet, dass** am Befestigungselement (4) wenigstens eine ein- oder beidseits hinterschnittene Nut (11) vorgesehen ist, in der das Funktionselement (5) aufgenommen ist.

8. Textilteil nach einem der Ansprüche 1 bis 6, **dadurch gekennzeichnet, dass** am Befestigungselement (4) wenigstens ein L-förmiger oder T-förmiger Vorsprung (26) ausgebildet ist, der in einer einseitig oder beidseits hinterschnittenen Nut (27) am Funktionselement (5) aufgenommen ist.

9. Textilteil nach einem der vorangehenden Ansprüche, **dadurch gekennzeichnet, dass** am Befestigungselement (4) eine oder mehrere Durchbrechungen (25) vorgesehen sind.

10. Textilteil nach einem der vorangehenden Ansprüche, **dadurch gekennzeichnet, dass** das oder die Funktionselemente (5) und das oder die Befestigungselemente (4) in einem Kunststoffspritzverfahren gefügt sind.

11. Textilteil nach einem der vorangehenden Ansprüche, **dadurch gekennzeichnet, dass** der Grundkörper (2) ein Gestrickkörper ist.

12. Textilteil nach einem der vorangehenden Ansprüche, **dadurch gekennzeichnet, dass** es eine Bandage (17), eine Orthese (18) oder ein Strumpf (23), an der oder dem ein längliches oder gebogenes stabartiges Funktionselement (5), ein gelenkartiges Funktionselement (19), ein als Gurtdurchführung ausgebildetes Funktionselement, ein als manuell greifbare Zughilfe (24) ausgebildetes Funktionselement oder ein als Pelotte ausgebildetes Funktionselement vorgesehen ist.

13. Verfahren zur Herstellung eines Textilteils nach einem der vorangehenden Ansprüche, **dadurch gekennzeichnet, dass** auf einem textilen Grundkörper (2) wenigstens ein Funktionselement (5), das formschlüssig und stoffschlüssig mit wenigstens einem Befestigungselement (4) verbunden ist, angeordnet wird, wonach durch Energiezufuhr zwischen dem Befestigungselement (4) und dem Grundkörper (2) eine stoffschlüssige Verbindung erwirkt wird.

14. Verfahren nach Anspruch 13, **dadurch gekennzeichnet, dass** durch die Energiezufuhr auch eine stoffschlüssige Verbindung zwischen dem Funktionselement (5) und dem Befestigungselement (4) erwirkt wird.

15. Verfahren nach Anspruch 13 oder 14, **dadurch gekennzeichnet, dass** das Befestigungselement oder das Befestigungselement (4) und das Funktionselement (5) oder eine zwischen dem Befestigungselement (4) und dem Grundkörper (2) angeordnete Zwischenlage zur Erwirkung der Verbindung lokal aufgeschmolzen wird.

## Claims

1. Textile part, in particular textile orthopaedic aid, comprising a textile main body (2), and at least one functional element (5) which is made of plastics material and disposed on the main body (2), wherein the functional element (5) is connected in a form-fitting and materially integral manner to at least one fastening element (4), wherein corresponding form-fit contours (9, 12) which engage with one another or span across one another are provided for establishing the form-fitting connection on the functional element (5) and on the fastening element (4), and the fastening element (4) is connected in a materially integral manner to the main body (2).

2. Textile part according to Claim 1, **characterized in that** the fastening element (4) is flexible or rigid.

3. Textile part according to Claim 1 or 2, **characterized in that** the fastening element (4) is a plastics-material element or has a carrier with a plastics-material coating on one side or both sides, or **in that** a fused intermediate tier which establishes the materially integral connection is provided between the fastening element (4) and the main body (2).

4. Textile part according to one of the preceding claims, **characterized in that** the functional element (5) and/or the fastening element (4) or the plastics-material coating or the intermediate tier are/is composed of polyamide, polyvinyl chloride, polyurethane, polyethylene or a thermoplastic elastomer.

5. Textile part according to one of the preceding claims, **characterized in that** further functional elements (5) are disposed either in a form-fitting manner or a form-fitting and materially integral manner on a common fastening element (4).

6. Textile part according to one of the preceding claims, **characterized in that** the functional element (5) is a functional element (5, 19) which is composed of a plurality of parts that are movable relative to one another, wherein one part is connected in a form-fitting and materially integral manner to a first fastening element (4), and a further part is connected in a form-fitting and materially integral manner to a second fastening element (4), and both fastening elements (4) are fastened in a materially integral manner to the main body (2).

7. Textile part according to one of the preceding claims, **characterized in that** at least one groove (11), which is undercut on one side or both sides and in which the functional element (5) is received, is provided on the fastening element (4).

8. Textile part according to one of Claims 1 to 6, **characterized in that** at least one L-shaped or T-shaped protrusion (26), which is received in a groove (27), which is undercut on one side or both sides, on the functional element (5), is configured on the fastening element (4).

9. Textile part according to one of the preceding claims, **characterized in that** one or a plurality of cut-outs (25) is/are provided on the fastening element (4).

10. Textile part according to one of the preceding claims, **characterized in that** the functional element or elements (5) and the fastening element or elements (4) are joined by a plastic injection-moulding method.

11. Textile part according to one of the preceding claims, **characterized in that** the main body (2) is a knitted fabric body.

12. Textile part according to one of the preceding claims, **characterized in that** said textile part is a bandage (17), an orthotic (18) or a stocking (23) on which is provided an elongate or curved rod-type functional element (5), a joint-type functional element (19), a functional element configured as a belt guide, a functional element configured as a lifting aid (24) which is able to be manually gripped, or a functional element configured as a support pad.

13. Method for producing a textile part according to one of the preceding claims, **characterized in that** at least one functional element (5), which is connected in a form-fitting and materially integral manner to at least one fastening element (4), is disposed on a textile main body (2), whereupon a materially integral connection is established by the input of energy between the fastening element (4) and the main body (2).

14. Method according to Claim 13, **characterized in that** a materially integral connection between the functional element (5) and the fastening element (4) is also established by the input of energy.

15. Method according to Claim 13 or 14, **characterized in that** the fastening element or the fastening element (4) and the functional element (5) or an intermediate tier disposed between the fastening element (4) and the main body (2) is/are locally fused in order to establish the connection.

## Revendications

1. Pièce textile, en particulier aide orthopédique textile, comprenant un corps de base textile (2) et au moins un élément fonctionnel (5) en matière synthétique disposé sur le corps de base (2), l'élément fonctionnel (5) étant relié par complémentarité de formes et une liaison de matière à au moins un élément de fixation (4), des contours à complémentarité de formes (9, 12) correspondants qui s'engagent l'un dans l'autre ou l'un par-dessus l'autre étant prévus sur l'élément fonctionnel (5) et sur l'élément de fixation (4) afin de réaliser la liaison par complémentarité de formes, et l'élément de fixation (4) étant relié par une liaison de matière au corps de base (2).

2. Pièce textile selon la revendication 1, **caractérisée en ce que** l'élément de fixation (4) est souple ou rigide.

3. Pièce textile selon la revendication 1 ou 2, **caractérisée en ce que** l'élément de fixation (4) est un élément en matière synthétique ou comporte un support pourvu d'un revêtement en matière synthétique sur une ou deux faces, ou **en ce qu'**une couche intermédiaire fondue, qui crée une liaison de matière, est prévue entre l'élément de fixation (4) et le corps de base (2) .

4. Pièce textile selon l'une des revendications précédentes, **caractérisée en ce que** l'élément fonctionnel (5) et/ou l'élément de fixation (4) ou le revêtement de matière synthétique ou la couche intermédiaire est en polyamide, polychlorure de vinyle, polyuréthane, polyéthylène ou un élastomère thermoplastique.

5. Pièce textile selon l'une des revendications précédentes, **caractérisée en ce que** d'autres éléments fonctionnels (5) sont disposés sur un élément de fixation commun (4) soit par une liaison par complémentarité de formes soit par une liaison par complémentarité de formes et une liaison de matière.

6. Pièce textile selon l'une des revendications précédentes, **caractérisée en ce que** l'élément fonctionnel (5) est un élément fonctionnel (5, 19) comprenant plusieurs parties mobiles les unes par rapport aux autres, une partie étant reliée par une liaison par complémentarité de formes et une liaison de matière à un premier élément de fixation (4) et une autre partie étant reliée par une liaison par complémentarité de formes et une liaison de matière à un deuxième élément de fixation (4) et les deux éléments de fixation (4) étant fixés par une liaison de matière au corps de base (2).

7. Pièce textile selon l'une des revendications précédentes, **caractérisée en ce qu'**au moins une rainure (11) en contre-dépouille sur un ou deux côtés, dans laquelle est reçu l'élément fonctionnel (5), est prévue sur l'élément de fixation (4).

8. Pièce textile selon l'une des revendications 1 à 6, **caractérisée en ce qu'**au moins une saillie (26) en forme de L ou de T est formée sur l'élément de fixation (4), laquelle saillie est reçue dans une rainure (27), en contre-dépouille sur un côté ou les deux côtés, sur l'élément fonctionnel (5).

9. Pièce textile selon l'une des revendications précédentes, **caractérisée en ce qu'**une ou plusieurs ouvertures traversantes (25) sont prévues sur l'élément de fixation (4).

10. Pièce textile selon l'une des revendications précédentes, **caractérisée en ce que** le ou les éléments fonctionnels (5) et le ou les éléments de fixation (4) sont assemblés dans un procédé de moulage par injection de matière synthétique.

11. Pièce textile selon l'une des revendications précédentes, **caractérisée en ce que** le corps de base (2) est un corps tricoté.

12. Pièce textile selon l'une des revendications précédentes, **caractérisée en ce qu'**il est prévu un bandage (17), une orthèse (18) ou un bas (23), sur lequel/laquelle est prévu un élément fonctionnel (5) allongé ou courbé en forme de tige, un élément fonctionnel (19) en forme d'articulation, un élément fonctionnel conçu comme un passage de ceinture, un élément fonctionnel conçu comme une aide à la traction (24) pouvant être saisie manuellement ou un élément fonctionnel conçu comme un coussin.

13. Procédé de réalisation d'une pièce textile selon l'une des revendications précédentes, **caractérisé en ce qu'**au moins un élément fonctionnel (5), qui est relié à au moins un élément de fixation (4) par une liaison par complémentarité de formes ou une liaison de matière, est disposé sur un corps de base textile (2), après quoi, par apport d'énergie entre l'élément de fixation (4) et le corps de base (2), une liaison de matière est créée.

14. Procédé selon la revendication 13, **caractérisé en ce que** l'apport d'énergie crée également une liaison de matière entre l'élément fonctionnel (5) et l'élément de fixation (4).

15. Procédé selon la revendication 13 ou 14, **caractérisé en ce que** l'élément de fixation ou l'élément de fixation (4) et l'élément fonctionnel (5) ou une couche intermédiaire disposée entre l'élément de fixation (4) et le corps de base (2) est fondu localement pour créer la liaison.
